# EUROPEAN PATENT SPECIFICATION

(11) **EP 3 368 886 B1**
(45) Date of publication and mention of the grant of the patent: **01.09.2021**
(21) Application number: 16858525.5
(22) Date of filing: 11.10.2016
(51) Int. Cl.: G01N 21/47, G01N 21/94, G01N 33/18

(54) **SENSOR FOR DETECTING REMOTELY LOCATED REFLECTIVE MATERIAL**
SENSOR ZUR ERKENNUNG VON ENTFERNT ANGEORDNETEM MATERIAL
CAPTEUR DE DÉTECTION DE MATÉRIAU RÉFLÉCHISSANT SITUÉ À DISTANCE

(30) Priority: 26.10.2015 US 201514756879
(43) Date of publication of application: 05.09.2018
(73) Proprietor: Adler, Jeffrey Scott, Beaconsfield, Quebec H9W 1R8 (CA); Baird, Harold, Russell, Marietta, GA 30064-1280 (US)
(72) Inventor: Adler, Jeffrey Scott, Beaconsfield, Quebec H9W 1R8 (CA); Baird, Harold, Russell, Marietta, GA 30064-1280 (US)
(74) Representative: Hertin und Partner Rechts- und Patentanwälte PartG mbB
(86) International application number: PCT/CA2016/000256
(87) International publication number: WO 2017/070772

(56) References cited:
- WO-A1-2014/022917
- JP-U- H 058 487
- US-A- 5 815 264
- US-A- 6 091 335
- US-A1- 2003 069 674
- US-A1- 2014 043 613

## Description

### TECHNICAL FIELD

The present invention relates to material sensors, and more particularly to a sensor for detecting remotely located reflective materials.

### BACKGROUND

Precipitation sensors have been developed to determine the presence of water in its vapor, liquid and solid forms, but usually the sensor is immersed in the material. Non-immersed sensing is a significant challenge. One example of a non-immersed sensor is the Bosch vehicle windshield rain sensor (Optical Sensor US Patent 6,376,824 by Michenfelder et al) used to operate windshield wipers. This sensor depends on the change in refraction of a reflected light beam against glass when water is on the outer glass surface. However, it has poor sensitivity for snow, unless the glass can be heated enough to melt the snow next to the glass. Moreover, it is unable to detect snow above the glass surface.

The ability to remotely detect reflective material at a distance away can be very valuable for numerous applications that encounter reflective material (such as winter precipitation or ice formed from super cooled water droplets) wherein there may be adverse effects encountered unless otherwise detected. Examples of which include detection of mid-air ice or super cooled water in the aerospace industry which would certainly welcome such an invention since ice detection has been a challenge for many years. An example of a sensor determining the presence of a material above a surface using reflection techniques is US 8741513 B2 Rain Sensor by Han. Disadvantageously, Han's sensor requires that the material be on the surface far side and be translucent so that the radiation can reflect off the material back surface through the translucent material in order to be sensed. Thus it will not detect clear ice which has a poor backside reflection. It is also susceptible to false signals such as sunlight, oncoming headlights, or street lamps shining through and mimicking a reflective signal. Han teaches that light sources and light receivers mounted on a plane inclined at an angle to the window prevent sources light from being reflected off the window surface back to light receivers. But drawing a complete set of light vectors on Han's art yields paths where source light is obviously reflected off the window surface to the light receivers, significantly reducing sensitivity to raindrop reflection signals. This loss of sensitivity is especially problematic, given that the majority of source light will pass through the raindrop rather than be reflected, and external light can also pass easily through the raindrop causing false signals to the light receivers. Han teaches the use of infrared light sources and receivers. Han's sensor specifically detects reflection from the far side of raindrops on a window surface. Han teaches a lattice pattern of light sources and receivers, suitable for the short distances in a windshield wiper control.

Another example US 8,873,062 or US 2014/043613 A1, respectively, by Adler & Baird is described as a sensor for sensing reflective material specifically on the transparent window surface of the sensor. The surface sensor is not able to detect reflective material remotely or at a distance. While the sensor does work well in terms of detection on the transparent surface, it does not have the capabilities to effectively detect away from the surface as provided for by the application herein that provides for the use of a radiation detector focusing lens and varied radiation emitters of differing intensities *and*/*or* radiation frequencies allowing for operational flexibility in numerous light conditions, to detect reflective materials at a distance away.

Another example is US 7285771 B2 Optical Sensor by Walker. Walker teaches the use of a single emitter and several detectors to determine paper position in a printer. While this sensor works well in the enclosed environment of a printer, with one emitter it does not have the flexibility to accommodate various light conditions found in other applications. Walker teaches using an infrared emitter and an enclosed housing to minimize the effect of stray light. This restricts the use to reflective material located within enclosed housing environments. Walker teaches an open air path for the radiated and reflected light. This is suitable for the benign environment in an enclosed housing in a printer.

In US 2003/0069674 A1, Stam et al. teach a moisture sensing system with radiation emitters, radiation emitter focussing lenses, a focusing lens that directs received radiation to a sensor comprising a subwindow array, and a window. Stam *et al.* imply the use of a housing around each emitter to prevent emitter radiation from being reflected from the window to the sensor. The reference further defines the sensor as an image sensor array and is associated with electronics required to perform image analysis in order to achieve windshield wiper, rear window defog and headlamp control.

In US 6,091,335, Breta et al. teach a single radiation emitter, radiation path mechanisms directing the radiation through a window to a point in space, a radiation reflection collector optical system, and various embodiment elements for detecting the polarization of the reflected radiation. Breta *et al.* do not teach any mechanism for preventing emitter radiation from being reflected from the window to the detectors.

Thus there is a need for a sensor useful in detecting remotely located reflective material.

### BRIEF SUMMARY

We have discovered through design, trial and error a remote reflective materials sensor that uses a reflective rather than refractive technique, and as such is very well suited to determining the presence of reflective materials located at a distance remote from a surface. Generally, a radiation source such as a Light Emitting Diode (LED) may be oriented to radiate at a distance through a transparent material such as glass. In one example, the transparent material is a window. When a reflective material such as snow or rime ice is within the sensor's field of view, a radiation detector such as but not limited to a phototransistor, photo diode or light dependent resister adjacent to the radiation source, may detect the radiation reflection.

According to a first aspect of the invention, there is provided a reflective materials sensor according to claim 1. According to a second aspect of the invention, there is provided a method of detecting reflective material according to claim 10.

The radiation emitters may be of differing intensities or radiation frequencies, allowing for operational flexibility in a variety of ambient light conditions and detecting a variety of reflective materials. Our discovery uses a mount, which also functions as a light baffle to prevent sensitivity degrading false reflections. Our device uses a variety of emitter and detector types, which can be modified depending on to the reflective material to be detected. Furthermore, according to the invention, our device uses *inter alia* detector path focusing lenses in the detector array embodiment that can extend the sensor range without increasing radiation emitter power. Moreover, our device may provide for a temperature sensor adjacent to the transparent window to distinguish between liquid and frozen reflective materials expected in a given application. While direct sensing of the transparent window temperature provides the best accuracy, alternate methods may be used. An example is a temperature sensor located elsewhere in the housing. Another example that may be used in a turbine engine application combines remote air temperature sensing from an aircraft fuselage mounted probe with algorithmic processing of engine parameters such as RPM to deduce engine temperature at the transparent window location. For example, engine air compression ratio at any compressor stage as a function of airspeed and engine RPM is determined in prototype development, or the engine may be instrumented with a pressure sensor. A thermodynamic equation can then be used to relate measured ambient air temperature to compressor stage temperature using compression ratio. Another example which may be used is an algorithmic only approach, whereby a combination of application operating parameters such as altitude and RPM can be used to deduce temperature at the transparent window position. Other methods may also be used, based on a person of ordinary skill in the art applying methods available in any given application.

Our device uses emitters aligned to a common target area outside the housing to produce a usable signal under varying ambient light conditions and reflective material locations outside the housing and away from it. We describe radiating through and receiving reflected light through a housing transparent window. This is suitable for a harsh environment such as detecting reflective material on a turbine engine compressor guide vane.

Furthermore, radiating through a transparent window allows the device to be used where the window isolates the sensor from harsh environments such as high or low temperatures or pressures, corrosive, toxic or flammable materials, and the like. The transparent window is also easier to clean than emitters and detectors. The transparent window material may also be selected to be transparent to the emitter *and*/*or* detector radiation wavelengths, but filter out undesirable ambient radiation.

In one example, the remote reflective materials sensor further includes a housing which houses a sensor mount, the radiation detector and the radiation emitters being mounted in the sensor mount. The sensor mount includes two spaced apart cavities aligned along the respective first axes in which the radiation emitters are located, and another cavity aligned along the second axis in which the radiation detectors are located.

In one example, the operating parameters sensor is selected from the group consisting of: a temperature sensor, a pressure sensor, an airspeed sensor, an RPM sensor, and an altitude sensor.

In one example, the radiation emitter is a Light Emitting Diode (LED).

In one example, the radiation emitter is an electroluminescent surface.

In one example, the radiation emitter is a narrow beam high radiation emitter selected from a laser, or a focused emitter, the focused emitter including a focused LED, a focused incandescent bulb, or a focused electric arc.

In one example, the radiation detector is a photo transistor, a photo diode or a light dependent resister located adjacent to the radiation emitter to detect reflected radiation.

In one example, the array of detectors is configured to detect spatially separated reflective material elements including individual snowflakes, ice crystals, or successive positions of one reflective object in the sensor field of view.

In one example, the first and second radiation emitters and the housing are configured so that radiation is emitted through the transparent window without causing a false radiation transparent window reflection back to the radiation detector.

In one example, a controller is located in the housing and is connected to a variable resistor, the radiation detector, the radiation emitter and the operating parameters sensor.

In one example, a controller is located in the housing and is connected to a fixed resistor, the radiation detector, the radiation emitter and the operating parameters sensor.

In one example, the radiation detector is an integrated circuit having a phototransistor, a photo diode or a light dependent resister located adjacent to the radiation emitter so as to detect reflected radiation.

In one example, the reflective material is winter precipitation. The winter precipitation is snow, sleet, frost, ice or ice pellets.

In one example, the reflective material is non-winter precipitation. The non-winter precipitation is reflective liquids, dirt, particulate material suspended in liquids, super cooled water droplets, or ice, including clear and rime ice.

According to another aspect, there is provided a use of the remote reflective materials sensor to detect reflective material located remote from the transparent window and associated with: airplanes, helicopters, drones, unmanned air vehicles, spacecraft, blimps, hybrid air/ground/marine/space vehicles, trucks, cars, motor bikes, recreational vehicles, trains, boats; sidewalks, driveways, walkways, roads, roofs, greenhouses, atriums, windows, skylights; food services, food preparation and preservation, freezer glass doors, freezers and/or refrigerators, buildings or infrastructure projects, medical applications including storage of tissues and cells, or sterilizations; landscaping including grass and garden maintenance, or crops weather determination, agriculture, climate, and ecosystem preservation; or energy production applications including solar applications for building materials including decking, walls or shingles.

In one example, the transparent window is made from a material that is transparent to emitter and detector radiation, and filters ambient radiation.

In one example, the operating parameters sensor is selected from the list of a temperature sensor, a pressure sensor, an airspeed sensor, an RPM sensor, and an altitude sensor.

### BRIEF DESCRIPTION OF THE DRAWINGS

In order that the discovery may be readily understood, embodiments are illustrated by way of example in the accompanying drawings.
**Figure 1** illustrates top view of a non-claimed remote reflective materials sensor;
**Figure 2** illustrates a side view of the remote reflective materials sensor showing radiation emitted and radiation reflected;
**Figure 3** illustrates the remote reflective materials sensor's field of view;
**Figure 4** is an embodiment according to the invention, illustrating the radiation detector array;
**Figure 5** is diagrammatic representation of communication between remote reflective materials sensor components in one example of the sensor; and
**Figure 6** is diagrammatic representation of communication between remote reflective materials sensor components in an alternative example of the sensor.

Further details of the device and its advantages will be apparent from the detailed description included below.

### DETAILED DESCRIPTION

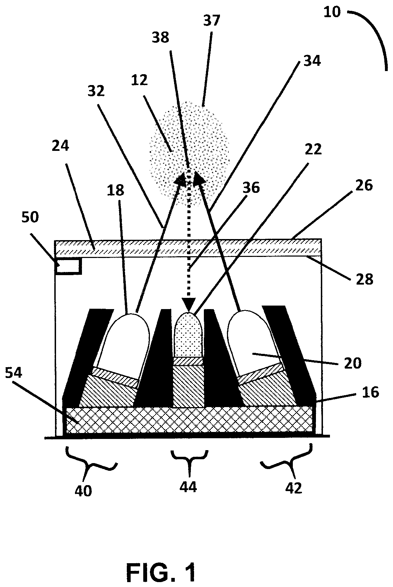

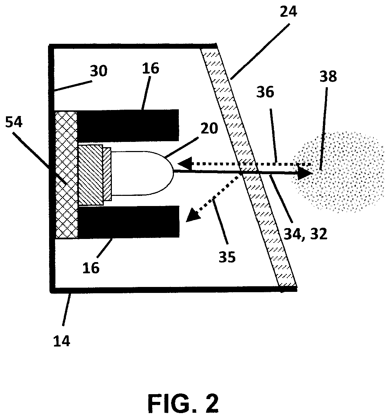

Referring to Figures 1, and 2, there is illustrated generally at 10 a remote reflective materials sensor for sensing reflective material 12 at a distance, a sensor outside the scope of the appended claims but useful for understanding the invention. In one example, the reflective material is winter precipitation such as, for example, snow, frost, ice or ice pellets. In another example, the reflective material is non-winter precipitation such as reflective liquids, dirt, super cooled water drops including clear and rime ice, or particulate material suspended in liquids. Broadly speaking, the remote reflective materials sensor 10 includes a housing 14, a sensor mount 16, two radiation emitters (radiation sources) 18, 20, a radiation detector 22 and a controller 54. The housing 14 has a transparent window 24 with first surface 26 and second surface 28, providing visibility of the reflective material 12 to the radiation emitters 18 and 20 and the radiation detector 22. The sensor mount 16 is located in the housing 14. The radiation emitters 18, 20 are mounted in the sensor mount 16. The radiation emitter 18, 20 each have a first axis 32, 34. Radiation is emitted from the radiation emitters 18, 20 along their respective axes 32, 34 towards and through the transparent window 24 until it contacts the distanced reflective material 12 away from the first surface 26 of the transparent window 24. The radiation detector 22 is mounted in the sensor mount 16 and adjacent and between the radiation emitters 18, 20. The radiation detector 22 and the radiation emitters 18, 20 are located away from the second surface 28 of the transparent window 24. The radiation detector 22 is located to receive the radiation that is reflected back from the distanced reflective material 12 along a second axis 36. The first axes 32, 34 of the radiation emitters 18, 20 are both angled towards the second axis 36. The two radiation emitters 18, 20 emit radiation towards a common focal point 38 on the distanced reflective material 12. The sensor mount 16 and window reflection path 35 prevent radiation emitter 18, 20 radiation from being mirror reflected to the radiation detector 22 by the transparent window 24. The radiation detector 22 is directed to the radiation emitter common focal point 38 on the distanced reflective material 12.

The temperature sensor only detects temperature. It does not detect barometric pressure or other parameters. It should be noted that the remote reflective materials sensor 10 will function without a temperature sensor. Without temperature, the reflective material sensor 10 assumes that any reflection is the reflective material ice of interest, and not dirt. With the temperature sensor, any reflection measured above freezing temperature can be assumed to be a foreign substance, allowing the implementation to trigger a maintenance operation by a technician.

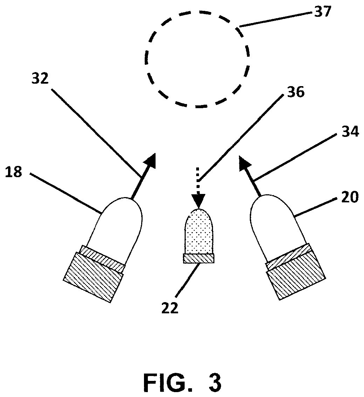

Referring briefly to Figure 3, radiation emitters 18 and 20 and radiation detector 22 have overlapping fields of useful radiation and detection to sense precipitation over area 37.

One skilled in the art will recognize that the reflective material sensor range can be extended by using narrow beam high radiation emitters such as, for example, lasers, focused LEDs, focused incandescent bulbs, or focused electric arcs.

Still referring to Figures 1, and 2, the sensor mount 16 includes two spaced apart cavities 40, 42 which are both aligned along their respective first axes 32, 34 in which the radiation emitters 18, 20 are located. Another cavity 44 is aligned along the second axis 36 in which the radiation detector 22 is located.

Still referring to Figure 1, a temperature sensor 50 is located adjacent to the transparent window 24 out of the radiation detector's 22 field of view, which will not cause a false reflection to the radiation detector.

By way of example each of the radiation emitters is a Light Emitting Diode (LED).

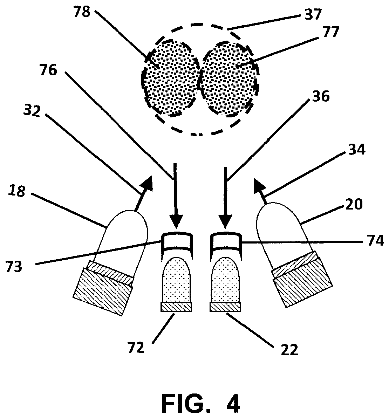

Referring now to Figure 4, an embodiment according to the invention is illustrated which is capable of detecting more than one reflective material element at a distance, and is therefore capable of detecting a moving or increasing or decreasing size of reflective material over time. Radiation detector 22 and at least one radiation detector 72 utitize focusing device 74 and at least one focusing device 73 to sense reflective material element 77 and at least one reflective material element 78.

Referring now to Figure 1, Figure 2 and Figure 5, a controller 54, which is typically a microprocessor or equivalent device, communicates with an included variable resister 56 or fixed resister 56A, the radiation detector 22, the radiation emitters 18, 20 and the temperature sensor 50 to achieve the reflective material sensing function. The controller 54 may be located within the housing 14, or in another suitable housing. One skilled in the art will understand that other devices and circuitry such as cabling, voltage supply, ground, signal buffering, user communication, controller programming, additional radiation detectors and associated variable or fixed resisters, and the like may also be integrated into the sensing function.

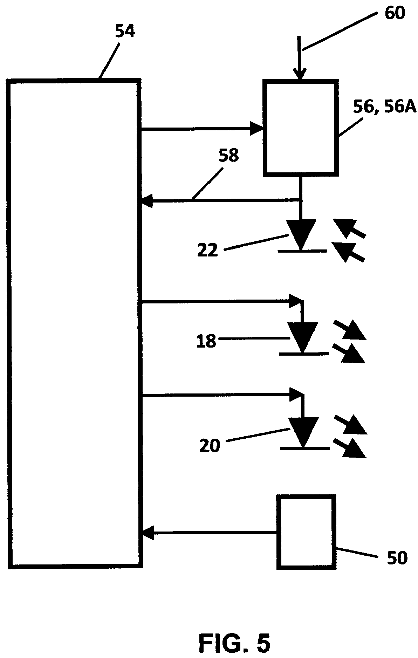

Referring now to Figure 5, the radiation detector 22 operates as an electrical current valve, which permits higher current flow at higher radiation levels. A reference voltage 60 is passed through the variable resister 56 and then the radiation detector to produce a radiation signal 58. As radiation increases, the current flow through the radiation detector 22 increases, causing an increased voltage drop across the variable resister 56. To allow for a wide range of radiation, the controller 54 modifies the value of the variable resister 56 to produce a usable signal. For installations where the ambient radiation range is small, an inexpensive fixed resister 56A may be used, thereby eliminating the need for the controller 54 to modify the resister 56A value. Alternatively, more than one copy of a fixed but different value resister 56A and radiation detector 22 may be used to broaden the sensed radiation range to provide maximum sensor detection of reflective material at a distance.

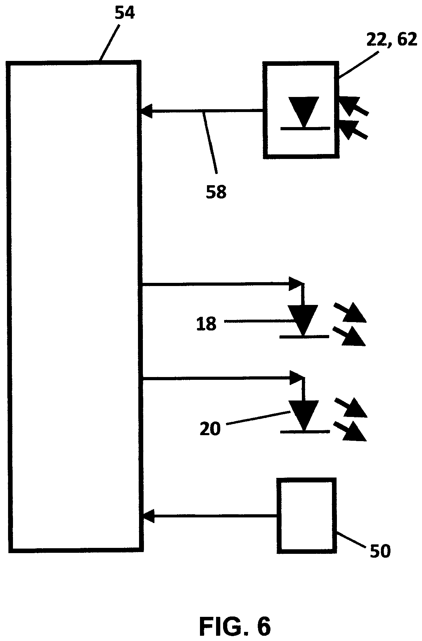

Referring now to Figures 1 and 6, the radiation detector 22 is an integrated circuit 62, which includes a radiation detector such as a photo diode, photo transistor or light dependent resister and a means to autonomously convert the radiation detector 22 output to the controller 54 compatible input such as frequency pulses.

Still referring to Figure 5 or 6, the controller 54 activates one or both of the radiation emitters 18, 20 when required to achieve the sense function. To assist in distinguishing between frozen and liquid reflective material, the controller 54 communicates with the temperature sensor 50 to determine whether frozen reflective material is possible.

The reflective material sensor 10 functions in a wide range of ambient radiations, from direct sunlight to nighttime. It can remotely sense reflective material on or at a distance away from for example, greenhouses, atriums, windows, freezer glass doors, skylights; on airplanes, drones, helicopters, spacecraft, aircraft, hybrid air/space/water/land vehicle components, and motorized transportation including trucks, cars, motor bikes, recreational vehicles, trains, boats and the like; food services, freezers /fridges, buildings, photovoltaic solar (conventional panels and non conventional solar applications), trough reflectors; for landscaping such as grass and garden maintenance, crops; or for weather determination, climate, ecosystem preservation; or for medical applications and storage of tissues and cells, sterilizations; or for food preparation and preservation, and the like. When operated in non-winter conditions, the remote reflective materials sensor 10 may also detect dirt on these types of surfaces to support cleaning operations. It can also detect ice crystal accretion in the atmosphere, which may not necessarily be associated with winter conditions. The remote reflective materials sensor 10 can also sense winter precipitation when installed in sidewalks, driveways, walkways, roads, roofs, infrastructure projects and the like. The remote reflective materials sensor 10 can be used in solar applications for building materials such as decking, walls and shingles.

While the remote reflective materials sensor 10 can be used to sense winter precipitation, it is easily applied to sensing other reflective materials such as, for example, liquids, precipitates, contamination, some gases, suspended solids, and the like, and as such can be applied to manufacturing and distribution processes for food, chemicals, fuels, and the like.

### Operation

Referring now to Figures 1, 2 and 5, operation of the remote reflective materials sensor 10 will be described. Distanced reflective material 12 is detected by determining the change in the radiation signal 58 when the radiation emitters 18, 20 are "off' then "on". Firstly, the controller 54 determines if reflective material 12 is possible by communicating with the temperature sensor 50 and any available external sources of data. If reflective material 12 is possible, then the controller 54 determines a reference ambient radiation signal 58 by first not switching on the radiation emitters 18, 20, then modifying the variable resister 56 until the radiation signal 58 is approximately 90% of the reference voltage 60. The controller 54 determines the reference ambient radiation by comparing the resultant variable resister 56 resistance with internally stored data. If the fixed resister 56A is used, the controller 54 determines reference ambient radiation by comparing the radiation signal 58 with internally stored data.

Referring now to Figure 6, an alternative operation of the remote reflective materials sensor 10 will now be described. Distanced reflective material 12 is detected by determining the change in the radiation signal 58 when the radiation emitters 18, 20 are "off" then "on". Firstly, the controller 54 determines if reflective material 12 is possible by communicating with the temperature sensor 50 and any available external sources of data. If reflective material 12 is possible, then the controller 54 determines a reference ambient radiation signal by first not switching on the radiation emitters 18, 20 then communicating with the radiation detector 62.

Referring now to Figures 5 and 6, the controller 54 then turns on one or both of the radiation emitters 18, 20 depending on the ambient radiation. At high ambient radiation, both radiation emitters 18, 20 may be required to obtain an adequate change in the radiation signal 58. The controller 54 then determines that reflective material 12 is present at a distance if the radiation signal 58 value has changed from the reference ambient radiation signal value by more than the combined effect of impurities in the transparent window 24 and expected dirt on the transparent window 24. The controller 54 may also determine the type of distanced reflective material 12 based on the combination of the temperature sensor 50 and the radiation signal 58 change, and the type of radiation emitter(s) and radiation detector(s) used.

In some applications such as low ambient light or reflective material easily detected at one radiation frequency, the dual emitter operations can be readily simplified for single emitter applications by anyone skilled in the art.

It should be noted that in the Figures, the area shown as the reflective material 12 is the sensor illumination or the detection coverage area. The reflected radiation signal will vary from a low value with no reflective material in the detection coverage area to a high value with highly reflective material covering the entire detection coverage area.

### Other Embodiments

From the foregoing description, it will be apparent to one of ordinary skill in the art that variations and modifications may be made to the embodiments described herein to adapt it to various usages and conditions, limited only by the appended claims.

## Claims

1. A reflective materials sensor (10) for detecting remotely located reflective material (12,77,78) within an area (37), the reflective materials sensor (10) comprising:
a transparent window (24) having first and second window surfaces (26 and 28);
an operating parameters sensor, for example a temperature sensor (50), and a controller (54) for executing an algorithm to determine a temperature of the transparent window (24), wherein the controller is in communication with the operating parameters sensor;
a first radiation detector (22) and at least one further radiation detector (72) located away from the second window surface (28);
first and second spaced apart radiation emitters (18 and 20) located on either side of radiation detectors (22, 72) and away from the second window surface (28), each radiation emitter (18, 20) being located to emit radiation along a first axis (32, 34), respectively, through the transparent window (24) towards reflective material in the area (37) located away from the first window surface (26), the radiation detectors (22, 72) being located to receive reflected radiation from the reflective material through the transparent window (24) along a second axis (36,76),
the first axes (32, 34) of the radiation emitters (18, 20) being angled towards the second axis (36,76) of the reflected radiation;
wherein the sensor further comprises a first lens (74) and one or more second lenses (73), and wherein the first lens (74) combined with the one or more second lenses (73) form an array arranged to focus reflected radiation from spatially separated reflective material elements (12, 77, 78) on an array of the first radiation detector (22) and the one or more further radiation detectors (72),
the radiation detectors (22,72) and the second axis (36) being in between the first and second radiation emitters (18, 20) and their first axes (32, 34) to form a common plane comprising the first and second axes (32,34,36);
and wherein the transparent window (24) is positioned to form a non-perpendicular angle to the common plane, thereby directing transparent window reflection axes (35) of radiation emitted along the first axes (32,34) and reflected at the window surface (28) away from the second axis (36), in order to prevent radiation from radiation emitters (18,20) from being reflected to the radiation detectors (22,72) by the transparent window (24).

2. The reflective materials sensor (10), according to claim 1, in which the first axes (32, 34) each being disposed at an acute angle toward the second axis (36), the common point (38) of the first axes (32, 34) and the second axis (36) converge on the reflective material (37) located away from the first window surface (26).

3. The reflective materials sensor (10), according to claim 1, in which the operating parameters sensor (50) is selected from the group consisting of: a temperature sensor, a pressure sensor, an airspeed sensor, an RPM sensor, and an altitude sensor.

4. The reflective materials sensor (10), according to claim 1, in which the radiation emitters (18, 20) are selected from the group consisting of: a Light Emitting Diode, LED, an electroluminescent surface, and a narrow beam high radiation emitter.

5. The reflective materials sensor (10), according to claim 5, in which the narrow beam high radiation emitter is a laser, or a focused emitter, the focused emitter including a focused LED, a focused incandescent bulb, or a focused electric arc.

6. The reflective materials sensor (10), according to claim 1, in which each of the radiation detectors (22, 72) is an integrated circuit light sensor, a photo transistor, a photo diode or a light dependent resistor located adjacent to the radiation emitter (18, 20) to detect reflected radiation.

7. The reflective materials sensor (10), according to claim 1, in which the radiation detectors (22, 72) are configured to detect spatially separated reflective material elements (77, 78) including individual snowflakes, ice crystals, or successive positions of one reflective object in the sensor field of view.

8. The reflective materials sensor (10), according to claim 1, in which a controller (54) is connected to a resistor (56, 56A), the radiation detectors (22, 72), the radiation emitters (18, 20) and the operating parameters sensor (50).

9. The reflective materials sensor (10), according to claim 1, in which the transparent window (24) is made from a material that is transparent to radiation emitter (18, 20) and radiation detector (22, 72) sensed radiation, and filters ambient radiation.

10. A method of detecting reflective material (12,77,78) remotely located away from a transparent window surface (26, 28), the method comprising, using the sensor of any of claims 1-9,
emitting radiation from the first and second radiation emitters (18, 22) each along a first axis (32, 34) through the transparent window (24) towards the reflective material (37);
receiving, by the radiation detectors (22,72), reflected radiation from the reflective material (37) along a second axis (36), the first axes (32, 34) of the radiation emitters being (18, 20) angled towards the second axis (36) of the reflected radiation;
wherein the method further comprises focussing the reflected radiation on the radiation detectors (22, 72) using the lenses (73, 74) disposed in the reflective material (12,77,78) to radiation detector (22, 72) pathway; and
inclining a radiation plane of the radiation emitter (18, 20) and radiation detector (22, 72) at a common non-perpendicular angle relative to a plane of the transparent window (24) so as to prevent radiation emitter (18, 20) axes radiation from being mirror reflected to the radiation detectors (22, 72) by the transparent window (24).

## Patentansprüche

1. Sensor (10) für reflektierende Materialien zur Erkennung eines entfernt angeordneten reflektierenden Materials (12, 77, 78) innerhalb eines Bereichs (37), wobei der Sensor (10) für reflektierende Materialien Folgendes umfasst:
ein transparentes Fenster (24), das eine erste und zweite Fensterfläche (26 und 28) aufweist;
einen Betriebsparametersensor, zum Beispiel einen Temperatursensor (50), und eine Steuerung (54) zum Ausführen eines Algorithmus, um eine Temperatur des transparenten Fensters (24) zu bestimmen, wobei die Steuerung mit dem Betriebsparametersensor in Kommunikation steht;
einen ersten Strahlungsdetektor (22) und mindestens einen weiteren Strahlungsdetektor (72), die entfernt von der zweiten Fensterfläche (28) angeordnet sind;
einen ersten und zweiten Strahlungsemitter (18 und 20), die voneinander beabstandet und auf jeder Seite der Strahlungsdetektoren (22, 72) und entfernt von der zweiten Fensterfläche (28) angeordnet sind,
wobei jeder Strahlungsemitter (18, 20) angeordnet ist, um Strahlung jeweils entlang einer ersten Achse (32, 34) durch das transparente Fenster (24) in Richtung des reflektierenden Materials in dem Bereich (37), der von der ersten Fensterfläche (26) entfernt angeordnet ist, zu emittieren, wobei die Strahlungsdetektoren (22, 72) angeordnet sind, um reflektierte Strahlung von dem reflektierenden Material durch das transparente Fenster (24) entlang einer zweiten Achse (36, 76) zu empfangen, wobei die ersten Achsen (32, 34) der Strahlungsemitter (18, 20) in Richtung der zweiten Achse (36, 76) der reflektierten Strahlung abgewinkelt sind;
wobei der Sensor ferner eine erste Linse (74) und eine oder mehrere zweite Linsen (73) umfasst und wobei die erste Linse (74) in Kombination mit der einen oder den mehreren zweiten Linsen (73) ein Array bildet, das eingerichtet ist, um reflektierte Strahlung von räumlich getrennten Elementen (12, 77, 78) aus reflektierendem Material an einem Array des ersten Strahlungsdetektors (22) und des einen oder der mehreren weiteren Strahlungsdetektoren (72) zu fokussieren,
wobei die Strahlungsdetektoren (22, 72) und die zweite Achse (36) zwischen dem ersten und zweiten Strahlungsemitter (18, 20) und ihren ersten Achsen (32, 34) liegen, um eine gemeinsame Ebene zu bilden, die die ersten Achsen und die zweite Achse (32, 34, 36) umfasst;
und wobei das transparente Fenster (24) positioniert ist, um einen nicht-rechten Winkel zu der gemeinsamen Ebene zu bilden, wodurch Reflexionsachsen (35) des transparenten Fensters von Strahlung, die entlang der ersten Achsen (32, 34) emittiert und
an der Fensterfläche (28) reflektiert wird, von der zweiten Achse (36) weg gelenkt werden, um zu verhindern, dass Strahlung von den Strahlungsemittern (18, 20) durch das transparente Fenster (24) zu den Strahlungsdetektoren (22, 72) reflektiert wird.

2. Sensor (10) für reflektierende Materialien nach Anspruch 1, bei dem die ersten Achsen (32, 34) jeweils in einem spitzen Winkel in Richtung der zweiten Achse (36) angeordnet sind, wobei der gemeinsame Punkt (38), an dem die ersten Achsen (32, 34) und die zweite Achse (36) an dem reflektierenden Material (37) konvergieren, von der ersten Fensterfläche (26) entfernt angeordnet ist.

3. Sensor (10) für reflektierende Materialien nach Anspruch 1, bei dem der Betriebsparametersensor (50) aus der Gruppe bestehend aus Folgenden ausgewählt ist: einem Temperatursensor, einem Drucksensor, einem Luftgeschwindigkeitssensor, einem U/min-Sensor und einem Höhensensor.

4. Sensor (10) für reflektierende Materialien nach Anspruch 1, bei dem die Strahlungsemitter (18, 20) aus der Gruppe bestehend aus Folgenden ausgewählt sind: einer Leuchtdiode (LED), einer elektrolumineszierenden Fläche und einem Emitter mit schmalem Strahl und starker Strahlung.

5. Sensor (10) für reflektierende Materialien nach Anspruch 5, bei dem der Emitter mit schmalem Strahl und starker Strahlung ein Laser oder ein fokussierter Emitter ist, wobei der fokussierte Emitter eine fokussierte LED, eine fokussierte Glühlampe oder einen fokussierten Lichtbogen beinhaltet.

6. Sensor (10) für reflektierende Materialien nach Anspruch 1, bei dem jeder der Strahlungsdetektoren (22, 72) ein Lichtsensor mit integrierter Schaltung, ein Fototransistor, eine Fotodiode oder ein lichtabhängiger Widerstand ist, der benachbart zu dem Strahlungsemitter (18, 20) angeordnet ist, um reflektierte Strahlung zu erkennen.

7. Sensor (10) für reflektierende Materialien nach Anspruch 1, bei dem die Strahlungsdetektoren (22, 72) dazu konfiguriert sind, räumlich getrennte Elemente (77, 78) aus reflektierendem Material zu erkennen, die einzelne Schneeflocken, Eiskristalle oder aufeinanderfolgende Positionen eines reflektierenden Objekts in dem Sichtfeld des Sensors beinhalten.

8. Sensor (10) für reflektierende Materialien nach Anspruch 1, bei dem eine Steuerung (54) mit einem Widerstand (56, 56A), den Strahlungsdetektoren (22, 72), den Strahlungsemittern (18, 20) und dem Betriebsparametersensor (50) verbunden ist.

9. Sensor (10) für reflektierende Materialien nach Anspruch 1, bei dem das transparente Fenster (24) aus einem Material gefertigt ist, das gegenüber erfasster Strahlung des Strahlungsemitters (18, 20) und des Strahlungsdetektors (22, 72) durchlässig ist und Umgebungsstrahlung filtert.

10. Verfahren zum Erkennen von reflektierendem Material (12, 77, 78), das entfernt von einer transparenten Fensterfläche (26, 28) angeordnet ist, wobei das Verfahren unter Verwendung des Sensors nach einem der Ansprüche 1-9 Folgendes umfasst:
Emittieren von Strahlung von dem ersten und zweiten Strahlungsemitter (18, 20) jeweils entlang einer ersten Achse (32, 34) durch das transparente Fenster (24) in Richtung des reflektierenden Materials (37);
Empfangen von reflektierter Strahlung von dem reflektierenden Material (37) entlang einer zweiten Achse (36) durch die Strahlungsdetektoren (22, 72), wobei die ersten Achsen (32, 34) der Strahlungsemitter (18, 20) in Richtung der zweiten Achse (36) der reflektierten Strahlung abgewinkelt sind;
wobei das Verfahren ferner Folgendes umfasst:
Fokussieren der reflektierten Strahlung an den Strahlungsdetektoren (22, 72) unter Verwendung der Linsen (73, 74), die in dem reflektierenden Material (12, 77, 78) angeordnet sind, zum Pfad des Strahlungsdetektors (22, 72);
und
Neigen einer Strahlungsebene des Strahlungsemitters (18, 20) und des Strahlungsdetektors (22, 72) mit einem gemeinsamen nicht-rechten Winkel relativ zu einer Ebene des transparenten Fensters (24), um zu verhindern, dass Strahlung der Achsen des Strahlungsemitters (18, 20) durch das transparente Fenster (24) zu den Strahlungsdetektoren (22, 72) spiegelreflektiert wird.

## Revendications

1. Capteur de matériaux réfléchissants (10) destiné à détecter un matériau réfléchissant (12, 77, 78) situé à distance dans une zone (37), le capteur de matériaux réfléchissants (10) comprenant :
une fenêtre transparente (24) ayant des première et seconde surfaces de fenêtre (26 et 28) ;
un capteur de paramètres de fonctionnement, par exemple un capteur de température (50), et un dispositif de commande (54) destiné à exécuter un algorithme afin de déterminer une température de la fenêtre transparente (24), dans lequel le dispositif de commande est en communication avec le capteur de paramètres de fonctionnement ;
un premier détecteur de rayonnement (22) et au moins un autre détecteur de rayonnement (72) situé à distance de la seconde surface de fenêtre (28) ;
des premier et second émetteurs de rayonnement (18 et 20) espacés situés de part et d'autre de détecteurs de rayonnement (22, 72) et éloignés de la seconde surface de fenêtre (28), chaque émetteur de rayonnement (18, 20) étant situé pour émettre un rayonnement le long d'un premier axe (32, 34), respectivement, à travers la fenêtre transparente (24) vers un matériau réfléchissant dans la zone (37) située à distance de la première surface de fenêtre (26), les détecteurs de rayonnement (22, 72) étant situés pour recevoir un rayonnement réfléchi provenant du matériau réfléchissant à travers la fenêtre transparente (24) le long d'un second axe (36, 76),
les premiers axes (32, 34) des émetteurs de rayonnement (18, 20) étant inclinés vers le second axe (36, 76) du rayonnement réfléchi ;
dans lequel le capteur comprend en outre une première lentille (74) et une ou plusieurs secondes lentilles (73), et dans lequel la première lentille (74) combinée avec les une ou plusieurs secondes lentilles (73) forment un réseau agencé pour focaliser un rayonnement réfléchi provenant d'éléments de matériau réfléchissant (12, 77, 78) séparés spatialement sur un réseau du premier détecteur de rayonnement (22) et des un ou plusieurs autres détecteurs de rayonnement (72),
les détecteurs de rayonnement (22, 72) et le second axe (36) se trouvant entre les premier et second émetteurs de rayonnement (18, 20) et leurs premiers axes (32, 34) pour former un plan commun comprenant les premier et second axes (32, 34, 36) ;
et dans lequel la fenêtre transparente (24) est positionnée pour former un angle non perpendiculaire au plan commun, dirigeant ainsi les axes de réflexion de fenêtre transparente (35) du rayonnement émis le long des premiers axes (32, 34) et réfléchi au niveau de la surface de fenêtre (28) à distance du second axe (36), afin d'empêcher le rayonnement provenant des émetteurs de rayonnement (18, 20) d'être réfléchi vers les détecteurs de rayonnement (22, 72) par la fenêtre transparente (24).

2. Capteur de matériaux réfléchissants (10) selon la revendication 1, dans lequel les premiers axes (32, 34) étant chacun disposés à un angle aigu vers le second axe (36), le point commun (38) des premiers axes (32, 34) et du second axe (36) convergent sur le matériau réfléchissant (37) situé à distance de la première surface de fenêtre (26).

3. Capteur de matériaux réfléchissants (10) selon la revendication 1, dans lequel le capteur de paramètres de fonctionnement (50) est sélectionné dans le groupe constitué : d'un capteur de température, d'un capteur de pression, d'un capteur de vitesse, d'un capteur de régime et d'un capteur d'altitude.

4. Capteur de matériaux réfléchissants (10) selon la revendication 1, dans lequel les émetteurs de rayonnement (18, 20) sont sélectionnés dans le groupe composé : d'une diode électroluminescente, DEL, d'une surface électroluminescente, et d'un émetteur à rayonnement élevé et à faisceau étroit.

5. Capteur de matériaux réfléchissants (10) selon la revendication 5, dans lequel l'émetteur à rayonnement élevé et à faisceau étroit est un laser ou un émetteur focalisé, l'émetteur focalisé comportant une DEL focalisée, une ampoule à incandescence focalisée ou un arc électrique focalisé.

6. Capteur de matériaux réfléchissants (10) selon la revendication 1, dans lequel chacun des détecteurs de rayonnement (22, 72) est un capteur de lumière à circuit intégré, un phototransistor, une photodiode ou une photorésistance situé à côté de l'émetteur de rayonnement (18, 20) pour détecter un rayonnement réfléchi.

7. Capteur de matériaux réfléchissants (10) selon la revendication 1, dans lequel les détecteurs de rayonnement (22, 72) sont conçus pour détecter des éléments de matériau réfléchissant (77, 78) séparés spatialement comportant des flocons de neige ou des cristaux de glace individuels ou des positions successives d'un objet réfléchissant dans le champ de vision du capteur.

8. Capteur de matériaux réfléchissants (10) selon la revendication 1, dans lequel un dispositif de commande (54) est relié à une résistance (56, 56A), aux détecteurs de rayonnement (22, 72), aux émetteurs de rayonnement (18, 20) et au capteur de paramètres de fonctionnement (50).

9. Capteur de matériaux réfléchissants (10) selon la revendication 1, dans lequel la fenêtre transparente (24) est composée d'un matériau qui est transparent au rayonnement détecté d'émetteur de rayonnement (18, 20) et de détecteur de rayonnement (22, 72), et filtre le rayonnement ambiant.

10. Procédé de détection de matériau réfléchissant (12, 77, 78) situé à distance d'une surface de fenêtre transparente (26, 28), le procédé comprenant, à l'aide du capteur selon l'une quelconque des revendications 1 à 9,
l'émission d'un rayonnement provenant des premier et second émetteurs de rayonnement (18, 22) chacun le long d'un premier axe (32, 34) à travers la fenêtre transparente (24) vers le matériau réfléchissant (37) ;
la réception, par les détecteurs de rayonnement (22, 72), d'un rayonnement réfléchi provenant du matériau réfléchissant (37) le long d'un second axe (36), les premiers axes (32, 34) des émetteurs de rayonnement étant (18, 20) inclinés vers le second axe (36) du rayonnement réfléchi ;
dans lequel le procédé comprend en outre
la focalisation du rayonnement réfléchi sur les détecteurs de rayonnement (22, 72) à l'aide des lentilles (73, 74) disposées dans le matériau réfléchissant (12, 77, 78) vers le chemin de détecteur de rayonnement (22, 72) ;
et
l'inclinaison d'un plan de rayonnement de l'émetteur de rayonnement (18, 20) et du détecteur de rayonnement (22, 72) à un angle non perpendiculaire commun par rapport à un plan de la fenêtre transparente (24) de manière à empêcher qu'un rayonnement d'axes d'émetteur de rayonnement (18, 20) soit réfléchi par miroir vers les détecteurs de rayonnement (22, 72) par la fenêtre transparente (24).
